# EUROPEAN PATENT APPLICATION

(11) **EP 3 804 526 A1**
(43) Date of publication of application: **14.04.2021**
(21) Application number: 20210848.6
(22) Date of filing: 07.04.2016
(51) Int. Cl.: A23C 9/123, A23C 9/12, C12N 9/38, C12P 7/26

(54) **FERMENTED MILK PRODUCT WITH DIACETYL PRODUCED WITH AID OF LACTASE**

(62) Divisional of application: 16164254.1
(71) Applicant: DSM IP Assets B.V., 6411 TE Heerlen (NL)
(72) Inventor: PRICE, Claire Emile, 6100 AA ECHT (NL)
(74) Representative: DSM Intellectual Property

(57) **Abstract**

The present invention relates to a method for producing a fermented milk product comprising diacetyl, which method comprises fermentation of a milk based substrate with lactic acid bacteria and the addition of a lactase enzyme, wherein the lactase enzyme contributes to the formation of diacetyl.

## Description

### Field of the invention

The present invention relates to a method for producing a fermented milk product comprising diacetyl. According to another aspect, the present invention relates to a fermented milk product comprising diacetyl. According to yet another aspect, the present invention relates to the use of a lactase enzyme for generating diacetyl in fermented milk products.

### Background of the invention

Lactic acid bacteria are extensively used for production of fermented foods, and they greatly contribute to flavor, texture and overall characteristics of these products. An old and well known example is yoghurt which probably originated from the Middle East and which still makes up more than half of the fermented milk production - or approximately 19 million tons in 2008 (source: Euromonitor). Fermented milks as e.g. yoghurts are popular due to the healthy image and pleasant sensory properties.

In many parts of the world an increasing interest in low fat fermented milk products is seen. This poses significant challenges for lactic acid bacteria culture as well as for the production process because it is difficult to produce low fat fermented milk products without reduction of sensory quality.

Yoghurt is generally produced from milk that has been standardized with respect to fat and protein content, homogenized and heat treated. Hereafter the milk is inoculated with a culture of *Streptococcus salivarius thermophilus* and *Lactobacillus delbrueckii* subsp. *bulgaricus* in large stirred fermentation or culturing tanks and subsequently fermented to a pH of around 4.6. In addition to the traditional yoghurt culture, a probiotic culture, as e.g. *Bifidobacterium,* can be applied to add extra health benefits.

Given the popularity of fermented milk products nowadays, there is an increasing demand for differentiating fermented milk products. For example fermented milk products having a distinct flavour profile without being the results of increasing amounts of sugar and / or fat. Therefore, there is a need in the art for alternatives to sugar and fat for generating a pleasant flavour profile to the fermented milk product.

### Summary of the invention

The objective of the present invention, amongst other objectives, is to provide a method for producing a fermented milk product comprising diacetyl.

This objective, amongst other objectives, is met by providing a method for producing a fermented milk product comprising diacetyl according to the appended claim 1.

Specifically, this objective is met by providing a method for producing a fermented milk product comprising diacetyl, which method comprises fermentation of a milk based substrate with lactic acid bacteria and the addition of a lactase enzyme, wherein the lactase enzyme contributes to the formation of diacetyl.

According to another aspect, the present invention relates to a fermented milk product comprising diacetyl in an amount of at least 5 mg/kg.

According to yet another aspect, the present invention relates to the use of a lactase enzyme for generating diacetyl in fermented milk products, preferably in yoghurt.

### Definitions

The term "milk based substrate" as used in the present context, may be any raw and/ or processed milk material. Useful milk based substrates may include solutions / suspensions of any milk or milk like products comprising lactose, such as whole or low fat milk, skim milk, buttermilk, reconstituted milk powder, condensed milk, solutions of dried milk, UHT milk, whey, whey permeate, acid whey or cream. Preferably the present milk based substrate is milk or an aqueous solution of skim milk powder.

The term "milk" is intended to encompass milks from mammals and plant sources or mixtures thereof. Preferably, the milk is from a mammal source. Mammals sources of milk include, but are not limited to cow, sheep, goat, buffalo, camel, llama, mare and deer. In an embodiment, the milk is from a mammal selected from the group consisting of cow, sheep, goat, buffalo, camel, llama, mare and deer, and combinations thereof. Plant sources of milk include, but are not limited to, milk extracted from soy bean, pea, peanut, barley, rice, oat, quinoa, almond, cashew, coconut, hazelnut, hemp, sesame seed and sunflower seed. Soy bean milk is preferred. In addition, the term "milk" refers to not only whole milk, but also skim milk or any liquid component derived thereof.

As used in this specification, the term "fermented dairy product" or "acidified dairy product" is intended to refer to products which are obtained by the multiplication of lactic acid bacteria in a milk base leading to a milk coagulum. The milk preparation used as raw material for the fermentation may be skimmed or non-skimmed milk, optionally concentrated or in the form of powder. Furthermore, this milk preparation may have been subjected to a thermal processing operation which is at least as efficient as pasteurization. The particular characteristics of the various fermented dairy products depend upon various factors, such as the composition of milk base, the incubation temperature, the lactic acid flora and/or non-lactic acid flora. Thus, fermented dairy products manufactured herein include, for instance, various types of regular yoghurt, low fat yoghurt, non fat yoghurt, kefir, dahi, ymer, buttermilk, butterfat, sour cream and sour whipped cream as well as fresh cheeses such as quark and cottage cheese.

As used in the present specification, the term "yoghurt" refers to products comprising "lactic acid bacteria such as *Streptococcus salivarius thermophilus* and *Lactobacillus delbruekii* subsp. *bulgaricus,* but also, optionally, other microorganisms such as *Lactobacillus delbruekii* subsp. *lactis, Bifidobacterium animalis* subsp. *lactis, Lactococcus lactis, Lactobacillus acidophilus* and *Lactobacillus casei,* or any microorganism derived therefrom. The lactic acid strains other than *Streptococcus salivarius thermophilus* and *Lactobacillus delbruekii* subsp. *bulgaricus,* are intended to give the finished product various properties, such as the property of promoting the equilibrium of the flora. "

As used herein, the term "yoghurt" encompasses set yoghurt, stirred yoghurt, drinking yoghurt, Petit Suisse, heat treated yoghurt and yoghurt-like products. Preferably, the yoghurt is a stirred yoghurt or a drinking yoghurt. More preferably, the yoghurt is a stirred yoghurt. The term "yoghurt" encompasses, but is not limited to, yoghurt as defined according to French and European regulations, e.g. coagulated dairy products obtained by lactic acid fermentation by means of specific thermophilic lactic acid bacteria only (i.e. *Lactobacillus delbruekii* subsp. *bulgaricus* and *Streptococcus salivarius thermophilus*) which are cultured simultaneously and are found to be live in the final product in an amount of at least 10 million CFU (colony-forming unit) / g. Preferably, the yoghurt is not heat-treated after fermentation. Yoghurts may optionally contain added dairy raw materials (e.g. cream) or other ingredients such as sugar or sweetening agents, one or more flavouring(s), fruit, cereals, or nutritional substances, especially vitamins, minerals and fibers. Such yoghurt advantageously meets the specifications for fermented milks and yoghurts of the AFNOR NF 04-600 standard and/or the codex StanA-IIa-1975 standard. In order to satisfy the AFNOR NF 04-600 standard, the product must not have been heated after fermentation and the dairy raw materials must represent a minimum of 70% (m/m) of the finished product.

In the present context, the terms "fresh cheese", "unripened cheese", "curd cheese" and "curd-style cheese" are used interchangeably herein to refer to any kind of cheese such as natural cheese, cheese analogues and processed cheese in which the protein/ casein ratio does not exceed that of milk.

The term "starter" or "starter culture" as used herein refers to a culture of one or more food-grade micro-organisms, in particular lactic acid bacteria, which are responsible for the acidification of the milk base. Starter cultures may be fresh (liquid), frozen or freeze-dried. Freeze dried cultures need to be regenerated before use. For the production of a fermented dairy product, the starter is usually added in an amount from 0.01 to 3%, preferably from 0.01 and 0.02 % by weight of the total amount of milk base.

As used herein, the term "lactic acid bacteria" (LAB) or "lactic bacteria" refers to food-grade bacteria producing lactic acid as the major metabolic end-product of carbohydrate fermentation. These bacteria are related by their common metabolic and physiological characteristics and are usually Gram positive, low-GC, acid tolerant, non- sporulating, non-respiring, rod-shaped bacilli or cocci. During the fermentation stage, the consumption of lactose by these bacteria causes the formation of lactic acid, reducing the pH and leading to the formation of a protein coagulum. These bacteria are thus responsible for the acidification of milk and for the texture of the dairy product. As used herein, the term "lactic acid bacteria" or "lactic bacteria" encompasses, but is not limited to, bacteria belonging to the genus of *Lactobacillus* spp., *Bifidobacterium* spp., *Streptococcus* spp., *Lactococcus* spp., such as *Lactobacillus delbruekii* subsp. *bulgaricus, Streptococcus salivarius thermophilus, Lactobacillus lactis, Bifidobacterium animalis, Lactococcus lactis, Lactobacillus casei, Lactobacillus plantarum, Lactobacillus helveticus, Lactobacillus acidophilus* and *Bifidobacterium breve.*

"Lactase" or "beta-galactosidase" (EC: 3.2.1.23) is an enzyme, which can convert lactose (disaccharides) into the monosaccharides glucose and galactose. Lactose is present in dairy products and more particularly in milk, skimmed milk, cream and other milk products. Lactases have been described for and isolated from a large variety or organisms, including micro-organisms. Lactase is often an intracellular component of micro-organisms like *Kluyveromyces* and *Bacillus. Kluyveromyces* and especially *K. fragilis* and *K.lactis,* and other yeasts such as those of the genera *Candida, Torula* and *Torulopsis* are a common source of yeast enzymes lactases, whereas *B. coagulans* or *B circulans* are well known sources for bacterial lactases. Several commercial lactase preparations, derived from these organisms are available such as Maxilact® (from *K. lactis,* produced by DSM, Delft, The Netherlands). All these lactases are so called neutral lactases since they have a pH optimum between pH = 6 and pH = 8.

Several organisms such as *Aspergillus niger* and *Aspergilus oryzae* can produce extracellular lactase, and US patent 5,736,374 describes an example of such lactase, produced by *Aspergillus oryzae.*

A lactase from *Bifidobacterium bifidum* has been described having a high transgalactosylating activity, both in the full-length form and especially when truncated from the C-terminal end (eg. see Jorgensen et al. 2001, Appl. Microbiol. Biotechnol., 57: 647-652 or EP1283876).

The enzymatic properties of lactases like pH- and temperature optimum vary between species. In general, however, lactases that are excreted show a lower pH-optimum of pH = 3.5 to pH = 5.0; intracellular lactases usually show a higher pH optimum in the region of pH = 6.0 to pH = 7.5, but exceptions on these general rules occur. The choice for a neutral or acidic lactase depends on the pH profile in the application. In applications with neutral pH, neutral lactases are usually preferred; such applications include milk, ice cream, whey, cheese, yoghurt, milk powder etc. Acid lactases are more suited for applications in the acidic range. The appropriate lactase concentration is dependent on the initial lactose concentration, the required degree of hydrolysis, pH, temperature and time of hydrolysis. The lactase of the present invention could be derived from all of the species mentioned.

Lactase activity may be determined as units of neutral lactase activity (NLU). In the present invention the lactase activity is determined using the method as described by the Food Chemicals Codex (FCC) IV method.

### Detailed description of the invention

In their research towards fermented milk products having improved flavour, the present inventors surprisingly found that the addition of a lactase enzyme in a process of fermentation of a milk based substrate, or milk, with lactic acid bacteria increases the amount of diacetyl formed in the fermented milk product. This is highly advantageous since diacetyl provides a pleasant buttery flavour to the fermented milk product. Moreover, increased amounts of diacetyl allow a further reduction of fat content in the fermented milk product, while maintaining the fat note of the product. It is further advantageous that the diacetyl is a naturally formed product and thus is not an additive which triggers labelling issues with the fermented milk product.

More specifically, the present inventors found a method for producing a fermented milk product comprising diacetyl, which method comprises fermentation of a milk based substrate, or milk, with lactic acid bacteria and the addition of a lactase enzyme, wherein the lactase enzyme contributes to the formation of diacetyl. The term 'Wherein the lactase enzyme contributes to the formation of diacetyl', as used in the present context means that the amount of diacetyl in the fermented milk product is higher if lactase enzyme has been added in comparison with fermented milk products produced without addition of lactase enzyme. In a preferred embodiment, the present invention relates to a method for producing a fermented milk product comprising diacetyl, which method comprises fermentation of milk with lactic acid bacteria and the addition of a lactase enzyme. More preferably, the present invention relates to a method for producing a fermented milk product comprising diacetyl, which method comprises fermentation of a milk based substrate, or milk, with lactic acid bacteria and the addition of a lactase enzyme, wherein diacetyl is produced, preferably wherein more diacetyl is produced than without addition of the lactase enzyme.

Preferably, the present lactic acid bacteria do not comprise *Lactococcus lactis subsp. lactis* biovar. *diacetylactis.* Strains of *Lactococcus lactis subsp. lactis* biovar. *diacetylactis* are known for their capability to provide diacetyl in fermented milk products. In the present invention however, the diacetyl formed is the result of adding lactase during manufacture. This is advantageous over using strains of *Lactococcus lactis subsp. lactis* biovar. *diacetylactis* since now the diacetyl production is on top of the desirable acidification, texture and product stability resulting from the lactic acid bacteria. Adding strains of *Lactococcus lactis subsp. lactis* biovar. *diacetylactis* with the purpose to generate diacetyl would dilute the effect of the other lactic acid bacteria.

In a preferred embodiment, the present invention relates to a method for increasing diacetyl levels in a fermented milk product, which method comprises fermentation of milk based substrate, or milk, with lactic acid bacteria and the addition of a lactase enzyme. Preferably, in such a method, the lactase enzyme contributes to the formation of diacetyl.

The amount of diacetyl in the fermented milk product is preferably at least 5 mg/kg diacetyl (5 mg diacetyl / kg fermented milk product), such as preferably at least 6 mg/kg diacetyl or more preferably at least 7 mg/kg diacetyl. Preferably, the amount of diacetyl in the fermented milk product is the amount present at the end of fermentation. The end of fermentation is for example once a pH of 4.6 is reached. The present inventors found that already directly at the end of fermentation the amount of diacetyl is increased after adding lactase enzyme during the manufacturing process.

An increase of diacetyl levels towards at least 5 mg/kg diacetyl is highly advantageous since the taste threshold for diacetyl is 0.03 mg/kg in homogenised milk. This means that each extra milligram of diacetyl significantly contributes to the flavour profile of the fermented milk product.

In a preferred embodiment, the amount of lactase enzyme is 50 to 10000 NLU/I milk based substrate or milk. More preferably the amount of lactase enzyme is 500 to 5000 NLU/I milk or 500 to 2500 NLU/I, or 2500 to 7500 NLU/I milk. More preferably the amount of lactase enzyme is an amount which is sufficient to increase the diacetyl level of the fermented milk product. Alternatively, the amount of lactase enzyme is an amount which is suitable to increase the diacetyl level of the fermented milk product. More preferably, the amount of lactase enzyme is an amount which is suitable to increase the diacetyl level of a fermented milk product, during storage of the fermented milk product.

In a preferred embodiment, the lactase enzyme is added to the milk based substrate, or milk, separately, simultaneously or sequentially with the lactic acid bacteria. Preferably, the present fermentation of milk with lactic acid bacteria is carried out in the presence of lactase. Thus preferably the lactase enzyme is added simultaneously with the lactic acid bacteria or before the lactic acid bacteria. Preferably, the initial lactase concentration in the milk is 50 to 5000 NLU/I. Alternatively, the lactase enzyme is added during fermentation of the milk with lactic acid bacteria. Thus, in this event, first the lactic acid bacteria are added to the milk and thereafter, during fermentation, the lactase enzyme is added. It is also envisaged to add the present lactase enzyme to the fermented milk product. Thus, in a preferred embodiment, the present method may comprise a step of addition of a lactase enzyme to the fermented milk product.

Surprisingly, the present inventors found that the amount of diacetyl increases during storage. Thus, diacetyl is not only formed during fermentation, but also after fermentation. This is advantageous in view of the stability and shelf-life of the product, since this allows a longer storage time for the product during its way from manufacturing to the consumer. Therefore, in a preferred embodiment, the present method further comprises a step of storing the fermented milk product. For example storing the fermented milk product for a time period of at least 5 or at least 7 days. More preferably storing at a temperature below 10°C, preferably below 7°C. Even more preferably, the present step of storing the fermented milk product increases the amount of diacetyl in the fermented milk product. More preferably the present method comprises a step of storing the fermented milk product to increase the amount of diacetyl in comparison with the amount of diacetyl measured at the end of fermentation. For example a step of storing the fermented milk product to increase the amount of diacetyl in comparison with the amount of diacetyl measured at the end of fermentation with an amount of at least 1 mg/kg fermented milk product, such as at least 2 or at least 3 or at least 5 mg/kg fermented milk product.

In a preferred embodiment, the present method comprises fermentation of the milk based substrate, or the milk, with lactic acid bacteria under thermophilic conditions. Preferably, the present method comprises fermentation of milk based substrate, or the milk, at a temperature within the range of 30°C to 45°C, more preferably 35°C to 45°C.

Surprisingly, the present inventors found that the addition of lactase under thermophilic conditions results in formation of diacetyl. This is unexpected since diacetyl formation is generally associated with mesophilic conditions. This is advantageous since thermophilic conditions allow a faster acidification.

Given the advantageous increased diacetyl amounts in the present fermented milk product, the present invention relates, according to another aspect, to a fermented milk product comprising diacetyl in an amount of at least 5 mg/kg such as preferably at least 6 mg/kg diacetyl or more preferably at least 7 mg/kg diacetyl.

In a further preferred embodiment, the present fermented milk product comprises diacetyl in an amount of at least 7 mg/kg after seven days after the end of fermentation, preferably at least 8 mg/kg after seven days after the end of fermentation. Surprisingly, the present inventors found that the amount of diacetyl increases during storage of the present fermented milk product. It is advantageous that the amount of diacetyl further increases in view of the pleasant buttery flavour which is provided to the fermented milk product. Preferably, the amount of diacetyl is measured after 7 days after the end of fermentation, while the fermented milk product was stored during 7 days at 6°C.

Preferably, the present fermented dairy product is yoghurt, drinking yoghurt, quark, Greek style yoghurt, kefir, dahi, ymer, buttermilk, butterfat, sour cream, creme freche, cream cheese, sour whipped cream or mozzarella. Preferably the present fermented dairy product is fermented milk, fresh cheese and / or fermented cream. Examples of are fresh cheeses, unripened cheeses or curd cheeses. Alternatively, the fermented dairy product is a ripened cheese.

In a further preferred embodiment, the present fermented milk product comprises lactic acid bacteria and / or a lactase enzyme. Preferably, the present fermented milk product does not comprise *Lactococcus lactis subsp. lactis* biovar. *diacetylactis.* Strains of *Lactococcus lactis subsp. lactis* biovar. *diacetylactis* are known for their capability to provide diacetyl in fermented milk products. In the present invention however, the diacetyl formed is the result of adding lactase during manufacture.

More preferably, the present fermented milk product is obtainable by the present method.

Given the beneficial diacetyl increasing properties of lactase enzyme, the present invention relates, according to another aspect, to the use of a lactase enzyme for generating diacetyl in fermented milk products, or the use of a lactase enzyme for providing a buttery flavor to fermented milk products. More preferably, the present lactase enzyme is used for providing a multi chocolate or a creamy, flavour to fermented milk products. Alternatively, the present invention relates to the use of the present lactase enzyme as fat replacement, as fat mimicking, or for providing a fatty flavour to fermented milk products.

Alternatively, the present invention relates to the use of a lactase enzyme for increasing the amount of diacetyl in fermented milk products during storage of the fermented milk product. Preferably during cold storage of the fermented milk product. More preferably during storage of the fermented milk product at a storage temperature below 10°C, preferably below 7°C and / or during storage of the fermented milk product for a time period of at least 5 days, such as at least 7 days. More preferably, the present invention relates to the use of a lactase enzyme for increasing the amount of diacetyl in fermented milk products during storage with at least 1 mg/kg fermented milk product, such as at least 2, at least 3 or at least 5 mg/kg fermented milk product.

The invention is further illustrated in the non limiting example below.

### Example 1

### Lactase addition to increase levels of diacetyl in yoghurt

### Methods

### Milk preparation

Reconstituted Skimmed Milk (RSM) was made using non-fat milk powder (DARIGOLD). The milk powder was added to room temperature water to reach an end concentration of 12% w/w and stirred for 15-30 minutes. The RSM was heat treated 100°C for 20 minutes.

### Yoghurt fermentation

The milk was pre-heated in a water bath to 40°C before cultures and/or lactase were added. Yoghurt and probiotic cultures used were from DSM Food Specialties BV, The Netherlands. DELVO® FRESH YS-341, DELVO® YOG FVV 121, DELVO® YOG FVV 122, VIABLE®LBC-1 and DELVO® PROLAFTI L26 were used at the dosages given in Table 1.

Where indicated, Maxilact LGi5000 was added to 5000 NLU per liter together with the yoghurt cultures.

**Table 1. Yoghurt cultures and culturing conditions used.**

| **Culture** | **Dosage** | **Fermentation temperature** | **Target pH at end of fermentation** |
|---|---|---|---|
| DELVO® FRESH YS-341 | 0,02% | 40°C | pH 4,6 |
| DELVO® YOG FVV 121 | 4U/1000L | 40°C | pH 4,6 |
| DELVO® YOG FVV 122 | 4U/1000L | 40°C | pH 4,6 |
| DELVO® PROLAFTI L26 | 4U/1000L | 37°C | <pH 4,4 |
| DELVO® YOG FVV 122 + DELVO® PROLAFTI B94 | 4U/1000L + 0,003% | 40°C | pH 4,6 |
| DELVO® YOG FVV 122 + VIABLE® Bif6 | 4U/1000L + 0,003% | 40°C | pH 4,6 |

Fermentation volumes were 250 ml and acidification was measured using the CINAC system from Alliance Instruments. Fermentations were stopped once a pH of 4,6 was reached. The yoghurts were then cooled immediately by immersion in an ice-cold water bath.

### Diacetyl levels in end product

Once cooled, the samples were placed at 6°C for a duration of 7 days. The diacetyl levels in the yoghurt were measured at one day and 7 days after fermentation by GC-MS. For the GC-MS, an internal standard solution was prepared by dissolving of 25 µl 3-methyl-2-pentanone in 500 ml acetonitrile using a volumetric flask. For the calibration standard, approximately 50 mg diacetyl was accurately weighed (within 0.01 mg) into a 100 ml volumetric flask and dissolved in water.

For the standard addition calibration curves a representative sample was selected and seven 20 ml headspace vials with screw cap were filled with approximately 1 g of sample (accurately weighed within 1 mg). Then, 0, 10, 25, 50, 75, 100 or 125 µl of the calibration standard was added. When necessary, the calibration standard was diluted to ensure that the peak area of diacetyl in the sample are in the same range of the peak area of diacetyl in the standard addition calibration line. Subsequently, 4.0 ml of internal standard solution was added to each of the vials and they were vortexed for 30 s and centrifuged for 5 min at 2500 rpm. The upper layer was transferred into a GC injection vial.

For the samples, approximately 1 g was accurately weighed (within 1 mg) into a 20 ml headspace vial with screw cap and 4.0 ml of the internal standard solution was added. The samples were vortexed for 30 s and centrifuged for 5 min at 2500 rpm, after which the upper layer was transferred into a GC injection vial.

The samples were analysed in single fold using an Agilent 7890A gas chromatograph equipped with a combi PAL liquid sampler, a split/splitless injector and a flame ionization detector. The injector was operated in the split mode (split 1:20) with an injector temperature of 250 °C. The injection volume used was 1 µl. The column used was a 30 m x 0.25 mm i.d. fused-silica capillary column DB-624 (Agilent J&W) with a stationary phase thickness of 1.4 µm. Hydrogen was used as the carrier gas with a pressure of 100 kPa. The temperature of the column was initially set at 40 °C (3 min hold), followed by a linear temperature gradient of 26.67 °C/min to 240 °C, where it was held for 3.5 min. The detector temperature was set at 325 °C. Dionex Chromeleon software was employed for data processing.

For the quantification of diacetyl, the slope (b) of the standard addition calibration line was determined by least square linear regression. In this standard addition calibration line, the relative peak area (A) of each standard addition solution was calculated by dividing the peak area of diacetyl by the peak area of the internal standard and this was plotted against the added amount of diacetyl in µg. The response factor (Rf) was calculated (Rf = 1/b). Furthermore, of each sample, the relative peak area (A) was calculated as described above, after which the concentration (C) can be calculated (C (µg/g) = A^{∗}Rf/Ms in which Ms is the weight of the sample in g).

### Results

The results are shown in table 2 below.

**Table 2. amounts of diacetyl**

| **Culture** | **Diacetyl (mg/kg)*** | | | |
|---|---|---|---|---|
| | With Maxilact | | No Maxilact | |
| | **Day 1** | **Day 7** | **Day 1** | **Day 7** |
| DELVO® YOG FVV 121 | 7 | 9 | 5 | 5 |
| DELVO® YOG FVV 122 | 7 | 8 | 5 | 5 |
| DELVO® YOG FVV 122 + VIABLE® Bif6 | 7 | 8 | 5 | 5 |
| DELVO® YOG FVV 122 + DELVO® PROLAFTI B94 | 7 | 9 | below detection limit | 5 |
| DELVO® FRESH YS-341 | 7 | 9 | 7 | 6 |
| DELVO® PROLAFTI L26 | 6 | 8 | 5 | below detection limit |

| | | | | |
|---|---|---|---|---|
| * the diacetyl concentrations are based on single measurements | | | | |

As clearly shown in table 2, in the yoghurts without Maxilact, the levels were in general close to the detection limit of 5 mg/kg, both at 1 day after fermentation and at Day 7. However, when Maxilact was added, the levels at Day 7 are consistently higher than those measured at Day 1 as well as those measured in the yoghurts without Maxilact at Day 7. Thus, the addition of Maxilact increasing the amounts of diacetyl in the yoghurt.

## Claims

1. Method for producing a fermented milk product comprising diacetyl, which method comprises fermentation of a milk based substrate with lactic acid bacteria and the addition of a lactase enzyme, wherein the lactase enzyme contributes to the formation of diacetyl.

2. Method according to claim 1, wherein the fermented milk product comprises at least 5 mg/kg diacetyl.

3. Method according to claim 1 or claim 2, wherein the fermented milk product comprises at least 5 mg/kg diacetyl at the end of fermentation.

4. Method according to any of the claims 1 to 3, wherein the amount of lactase enzyme is 50 to 5000 NLU/I milk.

5. Method according to any of the claims 1 to 4, wherein the lactase enzyme is added to the milk based substrate separately, simultaneously or sequentially with the lactic acid bacteria.

6. Method according to any of the claims 1 to 5, comprising fermentation of the milk based substrate with lactic acid bacteria under thermophilic conditions.

7. Fermented milk product comprising diacetyl in an amount of at least 5 mg/kg.

8. Fermented milk product according to claim 7, comprising diacetyl in an amount of at least 7 mg/kg.

9. Fermented milk product according to claim 7 or claim 8, further comprising lactic acid bacteria and / or a lactase enzyme.

10. Fermented milk product obtainable by the process according to any of the claims 1 to 6.

11. Use of a lactase enzyme for generating diacetyl in fermented milk products.

12. Use of a lactase enzyme for providing a buttery flavor to fermented milk products.

13. Use of a lactase enzyme for increasing the amount of diacetyl in fermented milk products during storage of the fermented milk product.
